# EUROPEAN PATENT APPLICATION

(11) **EP 0 707 865 A1**
(43) Date of publication of application: **24.04.1996**
(21) Application number: 95202824.9
(22) Date of filing: 18.10.1995
(51) Int. Cl.: A61M 29/02

(54) **Catheter with guide wire channel**

(30) Priority: 21.10.1994 NL 9401758; 15.02.1995 NL 9500283
(71) Applicant: CORDIS EUROPA N.V., NL-9301 LJ Roden (NL)
(72) Inventor: Dirks, Richard, NL-9403 KB Assen (NL); van Erp, Wilhelmus P.M.M., NL-9351 KS Leek (NL); Smit, Henderikus, NL-7848 CC Schoonoord (NL)
(74) Representative: 't Jong, Bastiaan Jacobus

(57) **Abstract**

The invention relates to a catheter comprising a tube-like basic body with a proximal and a distal end. The basic body comprises at least one lumen for receiving a guide wire, which extends from an end hole at the distal end to a wall hole in the wall of the basic body at a position in between the distal and the proximal end. The guide wire lumen is a lumen of a separate tube-like member which has been received in a lumen of the basic body, of which the relative proximal end is fixed within the wall hole.

## Description

The invention relates to a catheter which can be introduced into a patient by means of a guide wire. First a guide wire is introduced into the patient and advanced to the position to which an active element of the catheter is to be advanced. Next, the distal end of the catheter which is to be inserted is passed over the proximal end of the guide wire and advanced further along this guide wire, whereby the active element is advanced to the desired position inside the body of the patient.

With the catheter according to the invention as characterised in claim 1 such a catheter is obtained which is easy to manufacture and which is relatively thin and in which the guide wire lumen extends as a smooth and continuous surface from the end hole to the wall hole, so that inserting the guide wire into the guide wire lumen does not cause any problems. The catheter according to the invention can furthermore be manufactured relatively easy from separate tube-like elements with a single lumen. Complicated multi-lumen profiles are consequently superfluous.

Preferably the measure as set out in claim 2 is employed. As a result, it is in fact only the extreme end-section of the catheter which is guided and not its entire basic body.

A suitable, easy to manufacture embodiment is characterised in claim 3.

A further developed embodiment, whereby the transition from the inside to the outside of the catheter occurs very evenly is characterised in claim 4.

The measure as set out in claim 5 is preferably employed in order to achieve minimal resistance to movement along the guide wire and to obtain an outside catheter surface which is as smooth as possible.

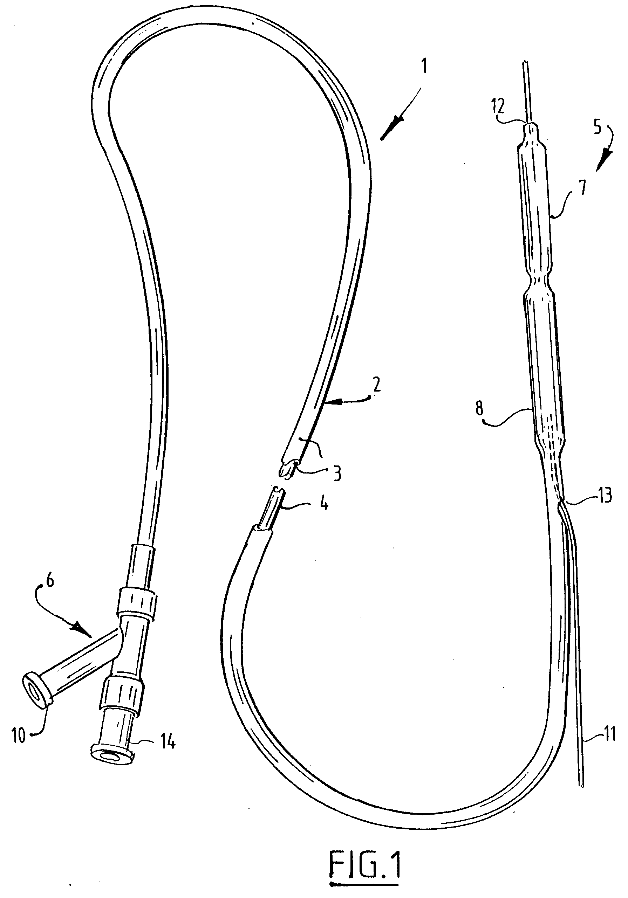

The invention will be explained in greater detail in the following description with reference to the attached drawings.
- Figure 1: shows a partly broken away perspective view of a catheter according to the invention.
- Figure 2: shows a partly broken away view of the end-section of the catheter of figure 1 when in use.
- Figure 3: shows a partly broken away view of an end-section of a catheter according to another embodiment.
- Figure 4: shows a partly broken away view of an end-section of yet another embodiment of the invention.
The catheter 1 shown in figure 1 comprises a tube-like basic body 2 with a distal end 5 and a proximal end 6. At the distal end 5 two balloons 7, 8 have been arranged. The balloons 7, 8 are for dilating a narrowed section of a blood vessel. The relatively proximal balloon 8 has a larger diameter in expanded situation, such that the dilation can be executed in two phases.

With the embodiment shown, the basic body 2 is made up of an outer tube-like element and an inner tube-like element 4. A lumen inside the inner tube-like element 4 has at the proximal end of the catheter 1 been connected to a connection 14, and at the distal end to the inside of the balloon 7 in order to convey medium under pressure from the connection 14 to the balloon 7 in order to expand the latter. In between the inner tube-like element 4 and the outer tube-like element 3, a in cross-section circular lumen is formed which, at the proximal end 6 of the catheter, is connected to the connection 10 and at the distal end 5 to the inside of the balloon 8 in order to be able to expand the latter in a similar manner.

For introducing the catheter into a patient a guide wire 11 is employed in the usual manner. For the purpose of receiving this guide wire 11 a lumen 16 has been formed in the catheter 1 which extends from an end hole 12 to a wall hole 13 in the catheter. This wall hole has been arranged in the wall of the basic body 2 at a position in between the distal and the proximal end.

With the preferred embodiment shown in figure 1, the wall hole has been arranged close to the relatively proximal active element of the catheter 1, the balloon 8. The lumen 16 for receiving the guide wire 11 only extends therefore through the end-section of the catheter 1.

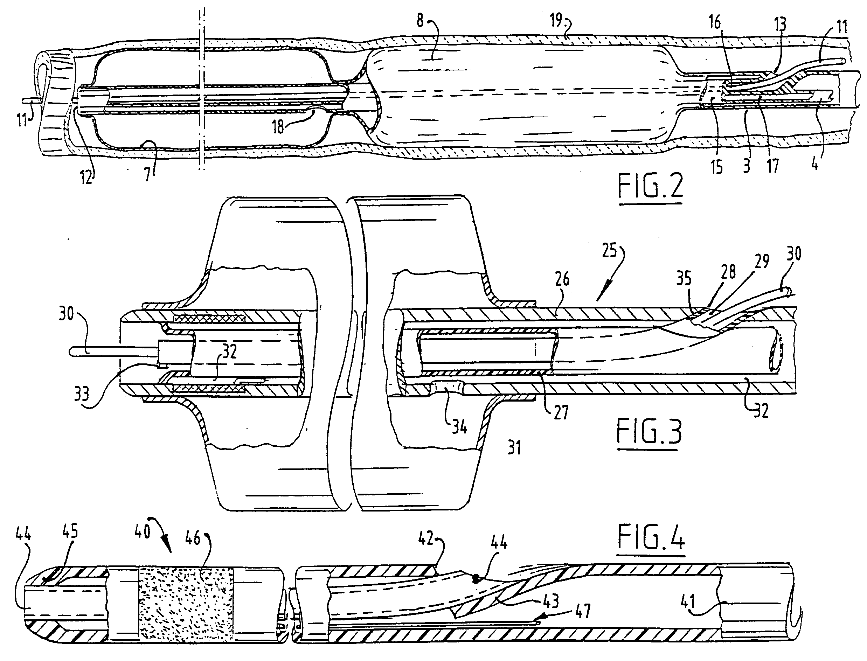

As figure 2 illustrates in greater detail, the guide wire lumen 16 has been formed inside a double-lumen element 15. The other lumen 17 of this element 15 is connected with the lumen of the inner tube-like element 4 of the basic body. The medium under pressure supplied via the connection 14 flows through the interspace between the double-lumen element 15 and the outer tube-like element 3 and, via the hole 18 in the wall of this double-lumen element 15, into the balloon 7 for dilating a narrowed section in the blood vessel 19.

The catheter 25 of figure 3 has another embodiment. The outer tube-like element 26 carries a balloon 31. In a central lumen of this outer tube-like element 26 an inner tube-like element 27 has been received through which for instance a contrast medium can be supplied. With this catheter 25, the lumen 29 for the guide wire 30 has been formed by the lumen of a separate tube-like element 28 which, with the end-section on the left-hand side as seen in figure 3, has been fixed at 33 close to the distal end of the catheter 25 and has been attached at the other end in an opening 35 in the outer tube-like element 26. During the manufacturing process the separate tube-like element 28 is pushed through the opening 35 until the end is situated at the end of the outer tube-like element 26. Subsequently the tube-like element 28 is fixed all round in the opening 35, for instance by glueing or ultrasonic welding. Next the protruding section of the tube-like element 28 is bevelled off and finished until it forms a smooth transition with the outer wall of the outer tube-like element 26.

With this embodiment the interspace 32 is formed in between the outer tube-like element 26 and the inner tube-like element 27 for supplying medium under pressure to the balloon 31 via opening 34 in the outer wall of the tube-like element 26 and for the discharge of medium from there. As can be seen in figure 2, the separate tube-like element 28 with the guide wire lumen 29 has also been passed through an opening in the wall of the tube-like element 27 and has preferably been sealed inside it.

Figure 4 shows a distal end-section of a catheter 40 which has been provided with an ultrasonic sensor 46 by way of active element.

Also in this case a separate tube-like element 44 has been employed of which the lumen forms the guide wire lumen of the catheter 40. At the distal end 45 the separate tube-like element 44 has been attached to the outer tube-like element 41 of the catheter 40 in such a manner as to form a seal, by for instance glueing or ultrasonic welding.

The other end of the separate tube-like element 44 has been fixed close to the wall hole 42 inside the tube-like element 41. The relatively proximal wall section 43 of the basic body formed by the tube-like element 41 has been folded inwards, and the relatively proximal end of the separate tube-like element 44 has been fixed on this inwardly folded wall section 43. As can be seen clearly in figure 4, the proximal wall section 43 of the basic body 41 forms a very even transition from the lumen of the separate tube-like section 44 to the outside. The basic body 41 is fixed all round to the proximal end-section of the separate tube-like element 44, thus creating a seal. The lines 47 connected to the ultrasonic sensor 46 extend through the central lumen of tube-like element 41 forming the basic body to the proximal end of the catheter 40, and are at that point connected to a processing device.

## Claims

1. Catheter comprising a tube-like basic body with a proximal and a distal end, wherein the basic body comprises at least one lumen for receiving a guide wire, extending from an end hole at the distal end to a wall hole in the wall of the basic body at a position in between the distal and the proximal end, and wherein the guide wire lumen is a lumen of a separate tube-like member which has been received in a lumen of the basic body, of which the relative proximal end is fixed within the wall hole.

2. Catheter as claimed in claim 1, comprising close to the distal end an active element like a sensor or a balloon, wherein the wall hole is situated close to the active element at that side of the wall hole turned away from the end hole.

3. Catheter as claimed in claim 1, wherein the wall hole is formed as a result of the fact that the relatively proximal end of the separate tube-like member protrudes through an opening in the wall of the basic body and has been fixed in that wall.

4. Catheter as claimed in claim 1, wherein the wall hole is formed as a result of the fact that the proximal wall section of the basic body has been folded inwards in the basic body close to an opening, and the relatively proximal end of the separate tube-like member has been fixed on the inwardly folded wall section.

5. Catheter as claimed in claim 3 or 4, wherein the relatively proximal end-section of the separate tube-like member has been bevelled off so as to merge into the wall section of the basic body around the opening therein.
